# EUROPEAN PATENT SPECIFICATION

(11) **EP 2 968 579 B1**
(45) Date of publication and mention of the grant of the patent: **15.04.2020**
(21) Application number: 14776545.7
(22) Date of filing: 07.03.2014
(51) Int. Cl.: A61K 9/14, A61K 9/16, A61K 9/20, A61K 47/36, A61K 47/30, A61K 47/26, A01N 25/10, A23L 33/10

(54) **STABLE BIOACTIVE SUBSTANCES AND METHODS OF MAKING**
STABILE BIOAKTIVE SUBSTANZEN UND VERFAHREN ZUR HERSTELLUNG
SUBSTANCES BIOACTIVES STABLES ET PROCÉDÉS DE FABRICATION

(30) Priority: 13.03.2013 US 201361779449 P
(43) Date of publication of application: 20.01.2016
(73) Proprietor: Intervet International B.V., 5831 AN Boxmeer (NL)
(72) Inventor: HAREL, Moti, Pikesville, MD 21208 (US); TANG, Qiong, Elkridge, Maryland 21075 (US); DICKEY, Erin, E., Laurel, MD 20723 (US)
(74) Representative: Intervet International B.V.
(86) International application number: PCT/US2014/021722
(87) International publication number: WO 2014/159051

(56) References cited:
- EP-A1- 0 695 173
- US-A- 4 063 017
- US-A- 4 997 599
- US-A1- 2005 181 952
- US-A1- 2006 147 494
- US-A1- 2011 195 101
- US-A1- 2012 039 956
- US-A1- 2012 121 717
- YUAN, W. ET AL.: 'Microencapsulation of protein-loaded polysaccharide particles within poly(D,L-lactic-co-glycolic acid) microspheres using S/O/W: characterization and release studies' POLYMERS FOR ADVANCED TECHNOLOGIES vol. 20, 2009, pages 834 - 842, XP055283843

## Description

### CROSS-REFERENCE TO RELATED APPLICATION

This application is related to and claims the benefit of U.S. Provisional Application No. 61/779,449 entitled "STABLE BIOACTIVE SUBSTANCES AND METHODS OF MAKING" filed on March 13, 2013.

### FIELD OF THE INVENTION

The present invention is in the field of stable bioactive materials, and the making and use thereof.

### BACKGROUND OF THE INVENTION

A common problem associated with the use of bioactive materials is the loss of activity due to oxidation, chemical decomposition during storage, product preparation, or in the animal's digestive tract before absorption. The harsh environment of some industrial processes, like milling, mixing, and pellet compression or extrusion, destroys a significant portion of the sensitive bioactive materials even before they become finished products. Additional problems resulted from the interaction between bioactive materials and other ingredients, such as metal chelators, strong surfactants, hygroscopic compounds, and etc. due to the sensitivity of bioactive materials, for example, enzymes to heat and surfactants (such as in feed extrusion and liquid detergents), or certain vitamins to oxidations and metal ions typically added to feeds, such as iron. Therefore, there is still a need for formulations and methods to protect and stabilize bioactive materials, including drugs, enzymes and vitamins, against decomposition during processing and shelf storage.

EP 0695173 discloses a process for loading bioactive substances onto polysaccharide particles.

### SUMMARY OF THE INVENTION

The present invention relates to stable bioactive substances and related preparation methods. The scope of the invention is defined by the claims.

According to a first aspect of the present invention, a method for preparing a stable bioactive substance comprising a bioactive material is provided. The method comprises (a) dissolving the bioactive material in a solution comprising solutes, wherein the solutes are a small molecule, selected from the group consisting of inorganic salts, and sugars, whereby a bioactive solution is obtained, wherein the bioactive solution has a bioactive material concentration of at least 5% and a solute concentration of less than 5% of the bioactive solution, each percentage based on the total weight of the bioactive solution; (b) adsorbing the bioactive solution onto dry non-gelling, water-soluble polysaccharide particles in a mass ratio of the bioactive solution to the polysaccharide particles between 0.25:1 and 1:1,whereby bioactive loaded polysaccharide particles are formed; and (c) drying the bioactive loaded polysaccharide particles, whereby the stable bioactive substance is obtained.

In some embodiments, the method further comprises removing a solute from the bioactive solution prior to the adsorption step.

In some other embodiments, the method further comprises adding an immiscible organic liquid to the stable bioactive substance, whereby a stable pourable suspension is formed.

In yet some other embodiments, the method further comprises coating the stable bioactive substance with a layer of molten solid fats or waxes.

In the method according to the present invention, the bioactive material may be selected from the group consisting of amino acid and their derivatives, drugs, peptides, enzymes, industrial enzymes, vitamins, vitamin derivatives and complexes, carotenes, antioxidants, anti-inflammatory substances, cosmetic oils, antimicrobial active ingredients, peroxides, agrochemicals and a mixture thereof. The polysaccharide may be selected from the group consisting of starches, celluloses, gums and their modified and derivative compounds.

According to a second aspect of the present invention, a stable bioactive substance prepared by the method of the present invention is also provided. The stable substance comprises a bioactive material in an amount ranging from 1% to 40% by weight and a polysaccharide in an amount ranging from 1% to 70% by weight. The bioactive substance may further comprise an emulsifier or stabilizer in an amount ranging from 0.5% to 10% by weight.

In the bioactive substance according to the present invention, the bioactive material may be an enzyme selected from the group consisting of proteases, lipases, amylases, cellulases, phosphatases, peroxidases, oxidases and a mixture thereof. The bioactive substance may further comprise a compound selected from the group consisting of natural or synthetic antioxidants, metal ions, alkali metal silicates, carbonates or bicarbonates, sulfates, phosphates, borates and boric acid. The compound may stabilize the enzyme.

According to a third aspect of the present invention, a product comprising the stable bioactive substance of the present invention is further provided.

In such an embodiment, the bioactive material is an enzyme. The product may be a textile product, carpet or surface cleaning product.

In another embodiment, the bioactive material is selected from the group consisting of a herbicide, an insecticide, a fungicide, a plant growth regulator, a seed treatment agent, and a mixture thereof. The product may be an agrochemical product.

In yet another embodiment, the bioactive material is selected from the group consisting of a vitamin, a protein, an enzyme, a mineral and a mixture thereof. The product may be selected from the group consisting of a feedstuff supplement, a food supplement, and a nutraceutical product.

In yet another embodiment, the bioactive material is selected from the group consisting of a drug compound, a bitter or aromatic compound, an antibiotic compound and a mixture thereof. The product may be a pharmaceutical product.

### DETAILED DESCRIPTION OF THE INVENTION

The present invention is based on the discovery of a novel method to prepare a stable bioactive substance. In particular, a solution substantially free of solutes is used to dissolve a bioactive material. A stable bioactive substance prepared using this novel method may be used to produce various products such as pharmaceutical, nutraceutical, foodstuff, agrochemical, cosmetics, personal hygiene and cleaning products.

A method is provided to prepare a stable bioactive substance. The method comprises dissolving a bioactive material in a solution substantially free of solutes to make a bioactive solution; adsorbing the bioactive solution onto dry polysaccharide particles to make bioactive loaded polysaccharide particles; and drying the bioactive loaded polysaccharide particles. In the absorption step, the mass ratio of the bioactive solution to the polysaccharide particles is between 0.25:1 and 1:1. The resulting stable bioactive substance may comprise 1-40% wt of a bioactive material and 1-70% wt of polysaccharide. One characteristic feature of the present invention is that the bioactive substance may be prepared without an substantial amount of solutes, for example, hydrogen bond donor solutes, which may interfere with chemical interactions between a bioactive material and a polysaccharide. The polysaccharide component is in the form of non-gelling flowable particles and onto which the bioactive material is absorbed. The presence of hydrogen-bond donor solutes may prevent the development of stabilizing interactions between the bioactive material and the polysaccharide. The non-gelling state of the polysaccharide particles provides essential hydrogen bonding interactions that stabilize the bioactive material and allow rapid dissolution of the bioactive material into water or upon consumption as compared with cross-linked polysaccharide hydrogel. The chemical interactions may preserve and/or maintain the bioactive material bound onto the polysaccharide particles. At the same time, the dry polysaccharide particles may also provide the needed melt flow and physical stability that is highly desirable in industrial applications. Various polysaccharides may be used to stabilize different bioactive materials because of their unique charging behavior and chemical interactions.

An adsorbing solution containing a bioactive material is substantially free of solutes, for example, low molecular weight solutes (e.g., electrolytes such as inorganic salts, sugars and solvents). Although some solutes may help solubilizing or enhancing the stability of a bioactive material in a solution, they may also interfere with more favorable stabilizing interactions between the bioactive material and the polysaccharide.

The stable bioactive substance may be quickly dissolved thereby releasing the bioactive material upon consumption or dilution in water. In a conventional gel dispersion system, the polysaccharide is already fully dissolved and the bioactive material is embedded in a droplet of harden or cross-linked polymers. Such gel systems are generally characterized by a porous structure, which may be insufficient for immobilizing and protecting the bioactive material from harmful temperatures and chemical attacks.

The polysaccharide may be either an anionic or cationic polymer depending on the type of the bioactive material to be used with. Hydrophilic bioactive materials such as water soluble pharmaceuticals, vitamins, chemicals, minerals and proteins may be dissolved in a water solution and adsorbed onto polysaccharide particles. The bioactive substance may be suspended in an immiscible liquid such as oil or hyper-osmotic liquid without forming a gel. Similarly, hydrophobic bioactive materials such as oil soluble vitamins, carotenes and essential oils may also be adsorbed onto polysaccharide particles. Optionally, the polysaccharide particles may be suspended in a hyper-osmotic liquid without forming a gel.

The polysaccharide particles containing the stable bioactive substance may be further coated for additional protection with a layer of molten solid fats, waxes, metal salts or a mixture thereof.

Polysaccharide particles containing heat and moisture stable enzymes may be useful in feed extrusion applications. An enzyme may be stabilized and its activity may be preserved by adsorbing onto polysaccharide particles without forming a gel and otherwise the enzyme may become inactivated or destroyed when extruded or mixed with other chemicals or feed ingredients at a typical effective dose.

Enzymes to be adsorbed onto polysaccharide particles may be those suitable for use in foodstuff, cosmetics and detergent compositions and are known in the art. Preferred enzymes may be proteases (e.g., subtilisin and savinase), lipases (e.g., Lipex and Lipolase),phosphatases (e.g., alkaline phosphatases), phosphodiesterases, or phytases. One or more enzymes may be included in a stable bioactive substance.

The bioactive substance may be in dry or liquid form, and may be further encapsulated or mixed with a stabilizer or inhibitor before mixed into a food, cleaning, or agrochemical product.

### DEFINITIONS

The term "polysaccharide" as used herein refers to a hydrophilic carbohydrate. It may be obtained from vegetable, animal, microbial, algal, fungal, yeast, bacterial, or synthetic origins, and is capable of absorbing a significant amount of water without loosening its granular structure. Suitable polysaccharides include, but are not limited to, gums such as agar, alginate, arabinoxylan, carrageenan, carboxymethyl cellulose, cellulose, chitosan, curdlan, gelatin, gellan, gelatin, β-glucan, guar gum, gum arabic, locust bean gum, lignin, pectin, xanthan gum, and starches (natural or modified).

The term "polysaccharide particles" as used herein refers to particles of any size, preferably having an average diameter from about 10 to about 1000 µm, comprising a polysaccharide in an amount of at least about 80% by weight, preferably at least about 90% by weight, more preferably at least about 99% by weight.

The term "bioactive loaded polysaccharide particles" as used herein refers to polysaccharide particles, onto which a bioactive material is absorbed. Bioactive loaded polysaccharide particles may be prepared by adsorbing a bioactive material in a solution substantially free of solutes onto dry polysaccharide particles.

The term "bioactive material" as used herein refers broadly any compound, or mixture thereof, suitable as an active ingredient in a pharmaceutical, nutraceutical, cosmetic, cleaning or agrochemical product and capable of producing a beneficial result in an environment to which the compound or mixture has been delivered. It may be either water-soluble or oil soluble. Examples of "bioactive materials" include proteins or peptides (e.g., enzymes, recombinant vaccines, antibodies, antimicrobial peptides, and the like), antibiotics, agrochemicals (e.g., pesticides, herbicides, germicides, biocides, algicides, rodenticides, fungicides, insecticides, and the like), antioxidants, plant and animal growth promoters, plant and animal growth inhibitors, preservatives, nutraceuticals, disinfectants, sterilization agents, catalysts, chemical reactants, fermentation agents, food or animal feed supplements, nutrients, flavors, colors, dyes, cosmetics, drugs, vitamins, sex sterilants, fertility inhibitors, fertility promoters, air purifiers, microorganism attenuators, nucleic acids (e.g., RNA, DNA, vectors, plasmids, ribozymes, aptamers, dendrimers, and the like), and other agents that provide improved activity at the site of action.

The term "enzyme" as used herein refers to a protein having a catalytic activity. It may be of animal, vegetable or microbial origin, or a chemically or genetically modified mutant. It may provide enzymatic performance in the environment or health benefits to a consuming organism. Exemplary enzymes include proteases, lipases, amylases, cellulases, phosphatases, peroxidases and oxidases.

The term "solute" as used herein refers to any small molecule capable of being dissolved in a solvent, for example, water or oil, to form a solution. Examples of "solutes" include inorganic salts, sugars, amino acids and short peptides. A short peptide has fewer than about 500, 200, 100, 50, 24 or 10 amino acids. A solution is substantially free of solutes where the solute concentration is less than 5% or 1% by weight.

The term "hydrogen-bonding donor" as used herein refers to a solute having a nitrogen, oxygen or sulphur atom which is covalently bound to at least one hydrogen atom. It may have a weak non-covalent chemical interaction with a bioactive material in a solution or with a polysaccharide.

The term "hyper-osmotic liquid" as used herein refers to a water solution containing at least about 50% by weight of an immiscible liquid such as oil, or a polyglycol, in which a polysaccharide is insoluble.

The term "stable" as used herein refers to the ability of a bioactive material or substance to retain, for example, at least about 80%, 90%, 95% or 99% of its functional activity at a temperature, for example, of about 0-100 °C, preferably about 2-40 °C, more preferably at room temperature, for an extended period of time, for example, at least about 1, 3, 12, 24 or 36 months, preferably at least about 3 months, more preferably at least about 12 months.

The term "heat stable" as used herein refers to the ability of a bioactive material or substance to retain, for example, at least about 80%, 90%, 95% or 99% of its functional activity when the temperature is increased by, for example, at least about 1, 5, 10, 20, 30, 50 or 100 °C.

In accordance with the present invention, a stable bioactive substance has been developed comprising a bioactive material and non-gelling polysaccharide particles. An adsorbing solution containing the bioactive material is substantially free of any solute and the bioactive material in the solution is adsorbed onto polysaccharide particles without cross-linking or forming gel matrices. A bioactive substance is useful in protecting a bioactive material from heat, moisture, destabilizing agents and oxidation damage. Preferably, a bioactive substance is rapidly dissolved to release a bioactive material upon dilution in water or upon consumption in the digestive tract of an animal. Preferable, the bioactive substance prevents premature release or de-activation of the bioactive material when mixed with other destabilizing agents.

The concentration of a bioactive material in a bioactive substance may vary within fairly wide limits, depending on its solubility, interaction with the polysaccharide and the intended use. For example, when the bioactive material is an enzyme, its concentration may be dictated in part by its desired specific activity upon consumption.

According to the invention, the bioactive material concentration is at least 5 %, based on the total weight of the bioactive substance, preferably at least about 10%.

Preferably the water-soluble polysaccharide is capable of absorbing a significant amount of water, for example, at least about 10%, 50%, 100%, 500% or 1000% of its dry mass, without dissolving and forming a gel or losing its fluid structure. Suitable polysaccharides may be obtained from natural, semisynthetic or synthetic origins. Natural organic materials are, for example, carrageenan, gum arabic, agar, agarose, guar gum, pectin, maltodextrins, alginic acid and its salts, e.g., sodium alginate or calcium alginate, collagen, chitosan, starches such those made from potatoes, maize, wax maize or high-amylose starch, wheat, rice or tapioca roots and also starch derivatives, in particular starch ethers and esters, dextran or cyclodextrins and chemically modified celluloses, in particular, cellulose esters and ethers, e.g., cellulose acetate, ethyl cellulose, hydroxypropyl cellulose, hydroxypropyl methyl cellulose and carboxymethyl cellulose,

Soluble gums and starches are particularly preferred since they are capable of absorbing water over 100% or more of their relative dry mass while still retaining flowability and granular structure. Various gums may be selected depending on their chemical or electrostatic interactions with the bioactive material and solubility in water and with respect to the specific application in order to obtain the desired protection during the industrial processes, storage and release upon consumption or in an environment. For example, alginate, pectin, guar gum or carrageen may be selected when the bioactive material is activated in ambient temperature while agar or xanthan gum may be selected when the bioactive material is activated at an elevated temperature. In general, polysaccharide concentrations of about 20% or more, based on the total weight of the bioactive substance are preferred, with concentrations of about 40% or more being more preferred.

The adsorbing solution containing a bioactive material may be substantially free of any hydrogen-bond donor solutes. The method of removing solutes from a bioactive material containing solution is not critical, and may be accomplished by any method known in the art such as precipitating the bioactive material, removing supernatant with precipitating solutes, dialyzing against water or a low strength buffer, or molecular sieving through a molecular cut-off membrane. Large quantities of various solutes such as salts, sugars and amino acids may remain in the solution along with the bioactive material. They may be needed during the production process of a bioactive material, for example, during fermentation, purification, concentration or stabilization. According to the present invention, those solutes may be substantially removed from the adsorbing solution in order to allow favorable chemical or electrostatic reactions between the bioactive material and the polysaccharide.

The bioactive loaded polysaccharide particles are dried to remove free water. Generally, suitable drying methods include air drying, vacuum or freeze drying and fluidized bed drying.

Optionally, the bioactive loaded polysaccharide particles may be coated with a layer of water repelling material. The bioactive in the coated bioactive loaded polysaccharide particles may be at least about 5%, 10%, 20%, 30%, 40%, 50%, 60%, 70%, 80%, 90%, 100%, 200%, 300% or 500%, preferably at least about 100%, more preferably at least about 200%, more active (e.g., stable) than that in the corresponding uncoated bioactive loaded polysaccharide particles. Preferred water repelling coating materials may be solid matrix-forming fats and waxes, including various thermoplastic materials such as natural or synthetic fatty alcohols, fatty acids, fatty esters and waxes. Natural waxes include vegetable waxes such as carnauba, candelilla, raffia, palm, sugar cane and cotton waxes; animal waxes such as beeswax and lanolin waxes and mineral wax such as paraffin and linear or branched polyethylenes, and microcrystalline waxes, and a mixture of various fats and waxes.

Optionally, the bioactive loaded polysaccharide particles may be suspended in a non-gelling liquid. Suitable suspension liquids include hydrophobic liquids, water-miscible organic liquids, or mixtures thereof. Suitable hydrophobic liquids include hydrocarbons like mineral oil, branched hydrocarbons like iso-paraffines, vegetable oils such as corn oil, peanut oil, soybean oil, and sunflower oil. Suitable water-miscible organic liquids include organic solvents such as ethylene glycol, propylene glycol, dicthylene glycol and glycerol.

The size of the bioactive loaded polysaccharide particles may vary, depending on other characteristics of the polysaccharide and use. In the case where a fast release of the bioactive is needed, the particles may be small enough to be readily dissolved upon dilution in water. Additional consideration may relate to the form of a final consumable product, which may be in a form free flowing powder, tablet, pellet or bar or in a form of a liquid product or suspension. Suitable particle sizes may be determined and produced using techniques known in the art. Generally, conventional mechanical size reduction, such as milling of the polysaccharide powder before loading a bioactive material, may be carried out where necessary to achieve a desired particle size. Further size reduction of the particles to sub-micron size may be achieved by subjecting a non-miscible suspension containing polysaccharide particles to high shear mixing using a conventionally high shear or ultra-high shear mixer. Preferably, at least about 90%, ideally all of the particles are smaller than about 1000 microns (diameter).In some applications where transparency or smooth texture is required, particles of about 50 microns or less are especially suitable.

The pH of an adsorbing solution containing a bioactive material may vary, as long as the pH is not detrimental to the bioactive material itself. The pH may be adjusted, as necessary, by adding an effective amount of a suitable acid or base. This may be done before loading the polysaccharide particles or after the addition of a stabilizing liquid, but is preferably done beforehand.

The bioactive substance of this invention may comprise an additional component such as a stabilizer, a preservative against the development of molds or bacteria in an effective to prevent degradation or auto-degradation of the bioactive material in the bioactive substance. The additional component may be present in any amount as long as it is in an amount sufficient to provide the desired stabilizing effect.

A stable bioactive substance may be produced by initially dissolving a hydrophilic bioactive material in an aqueous solution or weak buffered solution, or a hydrophobic bioactive material in an organic solvent or oil, where the solution, organic solvent and oil are substantially free of solutes. The resulting solution containing the bioactive material is herein after referred to as a "bioactive solution," and is substantially free of solutes. The concentration of solutes, including buffering salts, in a solution is less than 5%, preferably less than about 1%, based on the total weight of the bioactive solution. The concentration of the bioactive material is at least 5%, based on the total weight of the bioactive solution, preferably at least about 10%. The bioactive solution may be slowly poured and impregnated onto dry fine powder of desirable polysaccharide particles under gentle mixing until all the solution is absorbed by the polysaccharide particles. The mass (or weight) ratio between the bioactive solution and the dry polysaccharide is between 0.25:1 to 1:11, preferably about 0.5:1, and more preferable about 1:1.

It is generally desirable to continue stirring the polysaccharide dispersion while the bioactive material is absorbed and chemical/electrostatic interactions with the polysaccharide develop. Although the absorption may appear to be substantially completed, it is generally desirable to continue the stirring at the chosen reaction temperature for at least an hour or more, to give a maximum opportunity for full absorption and chemical/electrostatic interactions to develop. Stirring may then be discontinued and the resultant wet but still flowable composition may be either used as such for dispersing in immiscible liquid suspension or subjected to dehydration. Optionally, the polysaccharide particles may be added with one or more stabilizers or preservatives and/or lightly coated with a polymeric or solid fat layer.

Suitable immiscible liquid suspensions according to the present invention include those in which the bioactive material is substantially immiscible. For example, organic solvents are preferred for a protein bioactive, such as an enzyme or vaccine, while hydrophilic or aqueous liquids are better suited for hydrophobic or oil soluble bioactive, such as vitamin A or carotenes or essential oils (e.g., DHA, EPA, ARA, ALA). In general, the suspension liquid is added under continuous stirring until desirable flowability and viscosity is achieved. Preferably, the amount of the suspension liquid in the final composition is at least equal to the amount (by wt) of the bioactive loaded polysaccharide particles. However, any amount from as little as about 50% to over about 1000% relative to the bioactive loaded polysaccharide particles may be contemplated depending on the desired final viscosity and bioactive concentration.

The bioactive substance of present invention may be incorporated in a dry or liquid food composition. Non-limiting examples of such food products include non-carbonated and carbonated beverages, such as soft drinks, fruits or vegetables juices, beverages; sport drinks, energy drinks, drink-able yogurt, probiotic bacteria beverages or the like; dairy products; desserts such as yogurt and jellies, puddings, cream, mousse and the like, cold confections (e. g., ice cream and ice confections such as sherbets), hard candy, soft candy, flour pastes, whipped cream and the like; jams and the like.

The bioactive substance of present invention may be used as a drug in a pharmaceutical composition. Non-limiting examples of drugs include bitter aromatic compounds, analgesics, decongestants, antitussives, expectorants, antihistamines, mucolytics, laxatives, vasodilators, anti-arrhythmics, anti-diarrhea drugs, antihypertensives, antibiotics, narcotics, bronchodilators, anti-inflammatory drugs, cardiovascular drugs, tranquilizers, antipsychotics, antitumor drugs, sedatives, antiemetics, anti-nauseants, anti-convulsant, neuromuscular drugs, hypoglycemic agents, diuretics, antispasmodics, uterine relaxants, antiobesity drugs, antianginal drugs, and antiviral drugs and the like. Combinations of these drugs may also be used.

The bioactive substance of present invention may be used as a nutraceutical in a liquid or solid nutraceutical product. Non-limiting examples of nutraceuticals products include naturally nutrient-rich or medicinally active supplements, such as garlic, antioxidants, glucosamine, chondroitin sulfate; nutrients that provide health benefits, such as amino acids, vitamins, minerals, carotenoids, fatty acids such as omega-3 or omega-6 fatty acids, DHA, EPA, ARA, or ALA which can be derived from plant or animal sources (e.g., fish or algae), flavonoids, phenols, polyols, polyphenols, peptides, hydration agents, autoimmune agents or anti-inflammatory agents; or any other functional ingredient that can be delivered in a moist or liquid form and is beneficial to the prevention of specific diseases or conditions.

The bioactive substance of present invention may also be used as an agrochemical active ingredient in a liquid or solid agrochemical composition. Non-limiting examples of agrochemical active ingredients include herbicides, insecticides, fungicides, plant growth regulators and seed treatment agents. It is preferred that the agrochemical compositions are liquid compositions. The liquid agrochemical compositions may generally be applied to the target by spraying. The liquid agrochemical composition may be a suspension, for example, a concentrate suspension, which may be diluted with water prior to application. The bioactive substance of the present invention is readily soluble in water and generally compatible with water-soluble agrochemicals, and may be any of the commercial agrochemical active ingredients known in the art or listed in standard reference books such as the Pesticide Manual. A suitable agrochemical active ingredient may be paraquat, diquat, glyphosate, fomesafen, thiamethoxam, mesotrione, or trifloxysulfuron.

The bioactive substance of present invention may also be used to protect various active materials in textile, carpet or general surface cleaning liquids, such as enzymes, peroxides and bleaching agent compositions, and in particular enzymes in dry or liquid detergent. Non-limiting examples of cleaning compositions include dish soap, dish detergent, upholstery spot remover, laundry soap, laundry detergent, laundry stain remover, laundry additives, toilet bowl cleaner, soft scrubbing cleaner, and the like.

In one embodiment, a stable enzyme substance is produced by initially dissolving the enzyme in a solution substantially free of solutes, for example, an aqueous solution or weak buffered solution, or dialyzing solute-rich enzyme solution against weak buffer, to make an enzyme solution substantially free of solutes, which is hereinafter referred to as a solute free enzyme solution. The solute free enzyme solution is optionally added with an effective amount of an enzyme stabilizer or inhibitor. The solute free enzyme solution is then slowly adsorbed onto polysaccharide particles such as high purity , guar gum, pectin or carrageen powder under gentle mixing until uniformity is obtained, followed by removing free water by air drying, desiccation, vacuum or freeze drying or fluidized bed drying or any other known drying method. The bioactive loaded polysaccharide particles may optionally be coated with one or several layers of molten solid fats or waxes.

Preferably, the bioactive substance according to this invention is stable, for example, to the extent that it retains activity, by at least about 80%, 90%, 95% or 99%, in a consumable product for an extended period of time, for example, at least about 1, 3, 6, 12, 24 or 36 months, preferably at least about 3 months, more preferably at least 12, on standing at temperatures in the range from 2 to 40° C. More preferably, the bioactive substance is heat stable when the temperature is increased by at least about 1, 5, 10, 20, 30, 40, 50 or 100 °C.

The term "about" as used herein, when referring to a measurable value such as an amount, a percentage, and the like, is meant to encompass variations of ±20%, ±10%, more preferably ±5%, even more preferably ±1%, and still more preferably ±0.1% from the specified value, as such variations are appropriate.

The present invention will be further described with reference to the following Examples.

### EXAMPLE 1.

### Retention of hydrophilic bioactive materials in polysaccharide particles

Rhodamine 6G is a water-soluble chemical compound. It has a relatively small molecular weight (479.2 g/mol), and also readily soluble in a range of organic solvent such as glycerol and propylene glycol. It was used herein as a tracer dye to demonstrate its retention within polysaccharide particles suspension in organic solvent liquid.

**Preparation of cross-linked polysaccharide matrix particles, according to standard methods well known in the art:** A solution containing 10% rhodamine-6G (Sigma) in 1:1 mixture of water and propylene glycol was prepared. A water solution containing 3% wt carrageenan (Kappa-carrageenan gum, FMC BioPolymer, PA, USA)was prepared and 30% wt of the Rhodamine-6G solution was mixed in. The mixture containing the gum and the dye was sprayed in 10% potassium acetate water bath. It was evident that a significant amount of the dye already leached into the cross-linking solution. The gelled particles containing the remaining dye were collected, blotted on a paper towel and mixed in propylene glycol in 1:2 ratio of wet particles to the suspension organic liquid. After 1 h of gentle shaking at room temperature it was apparent that an additional significant amount of dye leached into the liquid suspension. This experiment demonstrates the difficulty of retaining small molecules in typical gel matrices.

**Preparation of a bioactive loaded polysaccharide particles according to the present invention:** Twenty five grams of highly purified and finely ground carrageenan powder was placed in a glass beaker. To that, twenty grams of the Rohdamine-6G solution was slowly added under continues mixing. Mixing continued until uniform and still flow-able wet powder was obtained. The dye-loaded particles were then placed in an air drier oven and dried for 1 h at 50°C. One hundred and twenty grams of propylene glycol was then added to the beaker and the suspension was mixed to uniformity. After 1-h of gentle shaking at room temperature it was apparent that the liquid remained clear and no apparent leaching of dye observed. This experiment demonstrates that the dye-loaded polysaccharide particles prepared according to the present invention can effectively retain small hydrophilic molecules in organic liquid suspension. To demonstrate the release of the dye upon dilution in water, 100 grams of water was added to 50 grams of the dye particle suspension. The liquid was immediately turned red, thereby confirming that the dye-loaded polysaccharide particles are readily releasing the small molecules upon dilution in water.

### EXAMPLE 2.

### Retention of hydrophobic bioactive materials in polysaccharide particles

Lucantin-Pink is a commercially available astaxanthin product manufactured by BASF, GmbH. Although astaxanthin is oil soluble chemical compound with relatively small molecular weight (596.84 g/mol), it has been made water dispersible in the product (see U.S. Pat. 7105176 [15]). It was used herein as a tracer pigment to demonstrate its retention within polysaccharide particles organic liquid suspension.

**Preparation of cross-linked polysaccharide particles, according to standard methods well known in the art:** A solution containing 10% Lucantin-Pink in 1:1 mixture of polypropylene glycol and mineral oil was prepared. A water solution containing 3% wt sodium alginate (FMC BioPolymer, PA, USA)was prepared and 25% wt of the Lucantin-Pink solution was mixed in. The mixture containing the polysaccharide and the pigment was sprayed in 5% calcium chloride water bath. It was observed that a significant amount of the pigment already leached into the cross-linking solution. The cross-linked particles containing the remaining pigment were collected, blotted on a paper towel and mixed in polypropylene glycol/mineral oil 1:1 w/w mixture in 1:2 ratio of wet particles in the organic liquid. After 1 h of gentle shaking at room temperature it was apparent that additional and significant amount of pigment leached into the organic liquid. This experiment demonstrates the difficulty of retaining small hydrophobic molecules in typical gel matrices.

**Preparation of a bioactive loaded polysaccharide particles according to the present invention:** fifty grams of finely ground sodium alginate was placed in a glass beaker. To that, 40 grams of Lucantin-Pink solution was slowly added under continues mixing. Mixing continued until uniform and still flow-able wet powder was obtained. The pigment-loaded particles were then placed in an air drier oven and dried for 2 h at 50°C. One hundred and fifty grams of polyethylene glycol/mineral oil 1:1 w/w mixture were then added to the beaker and the suspension was mixed to uniformity. After 1-h of gentle shaking at room temperature, the organic liquid remained substantially clear and no apparent leaching of dye observed while the polysaccharide particles retained their dark-colored and were easily visible in the liquid. This experiment demonstrates that the pigment-loaded polysaccharide particles, prepared according to the present invention, can effectively retain small hydrophobic molecules in either hydrophobic or organic solvent suspension. To demonstrate the release of the dye upon dilution in water, 100 grams of water was added to 50 grams of the pigment suspensions. The liquid was immediately turned red, thereby confirming that the pigment-loaded polysaccharide particles are readily releasing the small molecules upon dilution in water.

### EXAMPLE 3.

### Preparation of a heat stable polysaccharide substance containing enzyme.

Trypsin is a serine protease found in the digestive system of many vertebrates. It hydrolyses proteins and has substrate specificity based upon positively charged lysine and arginine side chains. Its molecular size is relatively small (24 kDa), which easily permeate polysaccharide gel matrices.

**Preparation of a stable enzyme polysaccharide substance according to the present invention:** A solution containing 10% wt of pure Trypsin powder (Sigma) in 0.01M Tris buffer is prepared. To this solution, 0.2% stabilizing agent (4-formylphenyl boronic acid, Sigma) is added. Guar gum (FMC BioPolymer, PA. USA) is finely ground in a kitchen coffee grinder and sieved through 50-micron mesh. Twenty five grams of finely ground guar gum is placed in a glass beaker. To that, twenty five grams of Trypsin solution is slowly added under continues mixing. Mixing continued until uniform and still flow-able wet powder is obtained. The enzyme-loaded particles are then placed in an air drier oven and dried for 1 h at 50°C. Fifty grams of polyethylene glycol are added to the beaker and the suspension homogenized for 15 minutes at 10,000 RPM (Tissue-Terror, Lab Homogenizer). The stable enzyme suspension containing about 1% trypsin is now ready for use in various liquid food or feed or nutraceutical products as described above.

**Heat stability testing of dry Trypsin substance in a tablet product:** Fifty mg of Trypsin substance containing 10% Trypsin is mixed with 450 mg of maltodextrin DE-1 containing 2% w/w magnesium stearate and 2% w/w hydrophilic fumed silica and compressed in hand held pill press equipment (using a ½" tablet diameter housing). Similar tablets containing dry powder of the enzymes in a free form is also prepared and used for comparison with tablets containing the heat stable enzymes substance.

The remaining activity of protease after tableting relative to their activity in the powder mix before tableting is determined according to methods known in the art using Azocasein as substrates. The protected and the control non protected Trypsin tablets are placed in an oven at 45°C and 75% relative humidity for 90 days. The remaining activity of Trypsin in both samples is measured following standard procedures [16]. No enzymatic activity is detected in the non-protected Trypsin containing sample, while the remaining activity in the sample containing Trypsin in stable substance is 70%.

### EXAMPLE 4.

**Preparation of a heat stable polysaccharide substance containing Phytase according to the present invention:** Phytase (Sigma) containing 2% wt active enzyme was dialyzed in 0.2M tris buffer to remove the added sugars and salts solutes from the enzyme solution. Twenty gram of the dialyzed enzyme solution was slowly added under continues mixing to twenty five grams of highly purified and finely ground powder of carrageenan, guar gum, agar, gellan or pectin in a glass beaker. Mixing continued until uniform and still flow-able wet powder were obtained. The Phytase loaded particles were then placed in an air drier oven and dried for 1 h at 40°C.

**Heat stability testing of a dry Phytase substance in simulated conditions of feed extrusion:** Five hundred mg of heat stable dry particles of the above Phytase substances were placed in a tube having a fine screen mesh bottom. The tube containing the Phytase substance was secured on top of an Erlenmeyer flask and subjected for 10 sec to a constant flow of hot steam produced by the boiling water in the flask. The remaining activity of the phytase after such simulated feed extrusion process was measured and compared with the remaining activity of free non-protected enzyme. Table 1 shows the remaining enzyme activity in each type of the polysaccharide carriers. It was found that all tested polysaccharides provided a significant protection to the enzyme under steam exposure for 10 sec. while no activity was detected in the non-protected enzyme sample. This test demonstrates that heat sensitive Phytase protected in polysaccharide substance according to the invention can be mixed with other animal feed ingredients and extruded to form feed pellets.

**Table 1. Phytase Activity in Particles under Simulated Feed Extrusion Conditions (steam exposure for 10 sec.)**

| **Adsorbing Carrier** | **Remaining Activity (%)** |
|---|---|
| Carrageen | 15% |
| Guar Gum | 26% |
| Agar | 32% |
| Gellan | 17% |
| Pectin | 39% |

### EXAMPLE 5.

### Effect of various carrier particles on the heat stability of Phytase in simulated feed extrusion conditions.

Dialyzed Phytase solution from Example 4 (20g) was adsorbed on 25 g corn starch, high amylose starch (Hylon V, National Starch and Chemical Co., Bridgewater, NJ), high purity Carrageen, Carboxymethyl cellulose (Hercules Inc., Wilmington, DE), Whey protein isolate (Davisco Food Int., Eden Prairie, MN), talc (sigma) and calcium carbonate (Sigma). Mixing continued until uniform and still flow-able wet powders were obtained. The Phytase loaded particles were then placed in a freeze drier and dried overnight at 40°C shelf temperature. The heat stability of the Phytase in the samples was tested in simulated feed extrusion conditions as described in Example 4 under steam exposure for 5 sec.). The remaining enzyme activity in the samples is given in Table 2. These testing results show that carrageen is the most suitable polysaccharide to protect Phytase in simulated feed extrusion conditions. Corn starch and inorganic carriers did not provide any protection to the enzyme.

**Table 2. Phytase Activity In Particles Under Simulated Feed Extrusion Conditions (steam exposure for 5 sec.)**

| **Adsorbing Carrier** | **Remaining activity (%)** |
|---|---|
| Corn starch | 0% |
| High amylose starch | 60% |
| Carrageen | 100% |
| CMC | 50% |
| Whey protein isolate | 60% |
| Talc | 0% |
| CaCo₃ | 0% |

### EXAMPLE 6.

### Heat stability in simulated feed extrusion conditions of protected Phytase in various polysaccharides and additional coat with a molten solid fat

Dialyzed Phytase solution from Example 4 (20g) was adsorbed on 25 g high purity Carrageen, guar gum (both from Tic gums, White Marsh, MD) agar, alginate or pectin (all from Sigma). Mixing continued until uniform and still flow-able wet powders were obtained. The Phytase loaded particles were then placed in a freeze drier and dried overnight at 40°C shelf temperature.

The dry phytase loaded polysaccharide particles were heated to 65°C and slowly added and mixed with an equal amount of molten hydrogenated soybean oil (27 Stereane, Loders Crokllan LLC., Channahon, IL). Magnesium or zinc stearate was added along with the molten fat to prevent clamping.

The heat stability of the Phytase in the coated samples was tested in simulated feed extrusion conditions as described in Example 4. The coated polysaccharide particles were subjected for 10 sec to a constant flow of hot steam produced by the boiling water in the flask. The remaining enzyme activity in the samples is given in Table 3. These testing results show that both guar gum and pectin are the most suitable polysaccharide to protect Phytase in simulated feed extrusion conditions.

**Table 3. Phytase Activity In Coated Particles Under Simulated Feed Extrusion Conditions (steam exposure for 10 sec.)**

| **Polysaccharide** | **Remaining activity (%)** |
|---|---|
| Carrageen | 30% |
| Guar gum | 70% |
| Pectin | 68% |
| Agar | 50% |
| Alginate | 30% |

### Example 7.

### Heat stability in simulated feed pelleting conditions of protected phytase in guar gum and additional coat with a molten solid fat

Dialyzed Phytase solution from Example 4 (20g) was adsorbed on 25 g high purity guar gum (Tic gums, White Marsh, MD). Mixing continued until uniform and still flow-able wet powders was obtained. The enzyme loaded particles were then placed in a freeze drier and dried overnight at 40°C shelf temperature.

The dry guar gum particles loaded with the enzyme were heated to 65°C and slowly added under mixing to an equal amount of molten hydrogenated soybean oil (27 Stereane, Loders Crokllan LLC., Channahon, IL). Magnesium or zinc stearate was added along with the molten fat to prevent clumping.

The heat stability of the enzyme particles was tested in simulated feed pelleting conditions where particle were mixed in milled chicken feed and subjected for 30 min to a constant flow of hot air at 85°C in an oven. The remaining enzyme activity in protected samples was 65% whereas the remaining activity in un-protected samples was only 32%.These testing results show that the protected Phytase according to the present invention is stable in simulated feed pelleting conditions.

### Example 8.

### Effect of various solutes in the adsorbing solution on heat stability of Phytase in simulated feed extrusion conditions.

Dialyzed Phytase solution from Example 4 (20g) was added with or without 10% calcium chloride and adsorbed on 25 g high amylose starch. Additional samples were prepared by adsorbing dialyzed Phytase solution from Example 4 (20g) added with or without 10% sucrose and adsorbed on 25 g low purity carrageen (containing a certain amount of maltodextrin). Dry Phytase substances were prepared as described in Example 4 and tested for heat stability under simulated feed extrusion conditions as described in example 4 under steam exposure for 5 sec.). The remaining enzyme activity in the samples is given in Table 4.These testing results demonstrate that solutes such as salts and sugars interfere with the positive interactions between polysaccharides and Phytase which was resulted in substantial lower enzyme activity after heat stability testing.

**Table 4. Heat Stability Under Simulated Feed Extrusion Conditions (steam exposure for 5 sec.)**

| **Solute and Carrier type** | **Remaining activity (%)** |
|---|---|
| No solute, High amylose starch carrier | 60% |
| 10% KCl, High amylose starch carrier | 30% |
| No solute, low purity carrageen carrier | 58% |
| 10% sucrose, low purity carrageen carrier | 8% |

### EXAMPLE 9.

### Preparation of stable polysaccharide substance containing an agrochemical.

Glyphosate (Monsanto) is a water-soluble organic compound and a widely used herbicide. It is a systemic, non-selective, post emergent herbicide, which is absorbed on foliage application through the plant to the roots. In a suspension form, this herbicide tends to rapidly lose activity, especially when mixed with other agrochemicals. This example demonstrates that Glyphosate protected in the stable polysaccharide substance of the present invention maintained a significant level of activity in organic liquid suspension in the presence of another herbicide agrochemical (Round-Up, Monsanto).

**Preparation of a stable agrochemicals substance according to the present invention:** Solutions containing Glyphosate or RoundUp (commercially available from Monsanto) in polypropylene glycol organic solution are prepared. To these solutions, 0.25% surfactant (Silicone-based nonionic surfactant, Kinetic™, Helena Corp) is added. Xanthan gum (FMC BioPolymer, PA, USA) is finely ground in a kitchen coffee grinder and sieved through 50-micron mesh. 100 grams of finely ground Xanthan gum is placed in a glass beaker. To that, 80 grams of Glyphosate or round-Up solutions are slowly added under continues mixing. Mixing continued until uniform and still flow-able wet powders are obtained. The beakers containing the herbicides-loaded particles are then incubated for 1 h at 50°C. Four hundred grams of mineral oil/polyethylene glycol mixture at 1:1 w/w ratio is then added to each beaker and the suspensions homogenized for 30 minutes at 10,000 RPM (Tissue-Terror, Lab Homogenizer). The stable herbicide concentrate suspensions are mixed together and are now ready for dilution in water before foliage application.

**Stability testing of the herbicides mixture suspension:** The commercial solutions containing Glyphosate or Round-Up are mixed together, and tested along with the stable Round-Up and Glyphosate mixed suspensions of the present invention. The liquids placed in an oven at 40°C for 30 days. The remaining activities of the herbicides in the samples are measured following standard protocols. No Glyphosate activity is detected in the free herbicides containing samples and only about 15% residual Round-Up activity remained, while the remaining activities in the samples containing protected Round-Up and Glyphosate mixed suspensions are 80% and 60%, respectively.

### EXAMPLE 10.

### Preparation of a stable polysaccharide substance containing vitamins supplement.

Vitamin B-12, also called cobalamin, is a water-soluble vitamin with a key role in the normal functioning of the brain and nervous system, and for generating red blood cells. A common synthetic form of the vitamin, cyanocobalamin, does not occur in nature, but is used in many pharmaceuticals and supplements, and as a food additive, because of its stability and lower cost. However, in tablet formulation, it becomes susceptible to degradation in the presence of other vitamins (e.g., Ascorbic acid) and minerals. This example demonstrates that Vitamin B-12 protected in the stable substance of the present is significantly more stable in the presence of Ascorbic acid.

**Preparation of a dry stable vitamins substance according to the present invention:** Solutions containing 10% wt pure vitamin B-12 or Ascorbic acid (Sigma) in 1:1 mixture of poly-propylene glycol and water are prepared. To these solutions, 0.25% emulsifier (Tween 80, Sigma) is added. Carboxymethyl cellulose (CMC, Sigma) is finely ground in a kitchen coffee grinder and sieved through 50-micron mesh. One hundred grams of finely ground CMC is placed in a glass beaker. To that, 80 grams Vitamin B-12 or Ascorbic acid solution is slowly added under continues mixing. Mixing continued until uniform and still flow-able wet powder is obtained. The drug-loaded particles are then placed in air drier oven and dried for 1 h at 40°C. The stable substances containing the vitamin B-12 and Ascorbic acid are mixed together and 500 mg tablets formed in a tablet press. Tablets are placed in 40°C and 75% RH desiccators for 3 months and remaining activity of both vitamins measured following standard protocols.

Various terms relating to the compositions, methods, and other aspects of the present invention are used throughout the specification and claims. Such terms are to be given their ordinary meaning in the art unless otherwise indicated.

### REFERENCES

1. Frankel E.N, et al., Oxidative stability of fish and algae oils containing long-chain polyunsaturated fatty acids in bulk and in oil-in-water emulsions. J Agric Food Chem., 2002. 50: p. 2094-9.
2. Vanlerberghe, G. Handjani N.V. Compositions comprising aqueous dispersions of lipid spheres, in US4897308. 1988.
3. Lykke, M. Mistry, K.K. Simonsen, O. Symes, K.C. Enzyme-containing particles and liquid detergent concentrate, in US6242405. 1998.
4. Seiler, M. Klee, S. Hills, G. Lersch, P. Smirnova, I. Arlt, W. Tschernjaew, J. Kobus, A. Encapsulation and Controlled Release of Biologically Active Ingredients with Enzymatically Degradable Microparticulate, Hyperbranched Polymers, in US Pat. App. 20080274149. 2008.
5. Maruyama, N., Y. Nishiyama, and H. Kokubo, Method of manufacturing a solid preparation coated with non-solvent coating, in US-5,789,014. 1996.
6. Kamel A, et al., Wax-encapsulated particles, in US5258132. 1993.
7. Soper J and Wampler D, Aqueous liquid flavor oil capsules, method of making and using in foods, in WO9319621. 1993.
8. Soper J, Pearl T, and Wampler D, Heat-stable and fracturable spray-dried free-flowing flavor oil capsules, method of making and using in foods, in WO9319622. 1993.
9. Lim F and Moss R, Vitamin encapsulation, in US4389419. 1983.
10. Cox, J.P., Method for forming shaped products for human and/or animal consumption or as marine bait and products produced thereby, in US4362748. 1982.
11. Weidenbach, G. and Bonse, D. Preparation of water-insoluble enzyme compositions, in US4230803. 1978.
12. Hawkins, J. Chadwick, P. Messenger, E.T and Lykke, M. Method for preparing stabilized enzyme dispersion, in US5198353. 1991.
13. Onouchi, T. Sugai, H. Sekiguchi, K. Hosoda, Y. Yoshida, E. Water-soluble microcapsules, in US. 4898781. 1987.
14. Langley, J. Symes, K.C. Holm, P. Methods of drying biological products. In US5035900. 1989.
15. Auweter, II. Bohn, H. Lüddecke, E. Hinz, W. Runge, F. Pfeiffer, A. Production of solid preparations of water-soluble, sparingly water-soluble or water-insoluble active compounds, in US7105176. 2001.
16. Bergmeyer, H.U., Gawehn, K., and Grassl, M. (1974) in Methods of Enzymatic Analysis (Bergmeyer, H.U., ed) Volume I, 2nd ed., 515-516, Academic Press, Inc.,New York, NY).

## Claims

1. A method for preparing a stable bioactive substance comprising a bioactive material,
comprising;
(a) dissolving the bioactive material in a solution comprising solutes, wherein the solutes are a small molecule, selected from the group consisting of inorganic salts, and sugars, whereby a bioactive solution is obtained, wherein the bioactive solution has a bioactive material concentration of at least 5% and a solute concentration of less than 5% of the bioactive solution, each percentage based on the total weight of the bioactive solution;
(b) adsorbing the bioactive solution onto dry non-gelling water-soluble polysaccharide particles in a mass ratio of the bioactive solution to the polysaccharide particles between 0.25: 1 and 1:1, whereby bioactive polysaccharide loaded particles are formed; and
(c) drying the bioactive polysaccharide loaded particles, whereby the stable bioactive substance is obtained.

2. The method of claim 1, further comprising removing a solute from the bioactive solution prior to the adsorption step.

3. The method of claim 1, further comprising adding an immiscible organic liquid to the stable bioactive substance, whereby a stable pourable suspension is formed.

4. The method of claim 1, wherein the bioactive material is selected from the group consisting of amino acid and their derivatives, drugs, peptides, enzymes, industrial enzymes, vitamins, vitamin derivatives and complexes, carotenes, antioxidants, anti-inflammatory substances, cosmetic oils, antimicrobial active ingredients, peroxides, agrochemicals and a mixture thereof.

5. The method of claim 1, wherein the polysaccharide is selected from the group consisting of starches, celluloses, gums and their modified and derivative compounds.

6. The method of claim 1, wherein the polysaccharide is carrageen, pectin, guar gum or carboxymethyl cellulose.

7. The method of claim 1, wherein the stable bioactive substance comprises the bioactive material in an amount of at least 1 % by weight and the polysaccharide in an amount of 20% or more by weight, each percentage based on the total weight of the bioactive substance.

8. The method of claim 1, further comprising coating the stable bioactive substance with a layer of molten solid fats or waxes.

9. The method of claim 1, wherein the mass ratio of the bioactive solution to the polysaccharide particles is 0.5: 1.

10. A stable bioactive substance comprising a bioactive material in an amount ranging from 1 % to 40% by weight and a water-soluble polysaccharide in an amount ranging from 1 % to 70% by weight, wherein the stable bioactive substance is produced by the method of claim 1.

11. The stable bioactive substance of claim 10, further comprising an emulsifier or stabilizer in an amount ranging from 0.5% to 10% by weight based on the total weight of the bioactive substance.

12. The stable bioactive substance of claim 10, wherein the bioactive material is an enzyme selected from the group consisting of proteases, lipases, amylases, cellulases, phosphatases, peroxidases, oxidases and a mixture thereof.

13. The stable bioactive substance of claim 12, further comprising a compound selected from the group consisting of natural or synthetic antioxidants, metal ions, alkali metal silicates, carbonates or bicarbonates, sulfates, phosphates, borates and boric acid.

14. The stable bioactive substance of claim 13, whereby the compound stabilizes the enzyme.

15. A product comprising the stable bioactive substance of claim 10, wherein the bioactive material is an enzyme.

## Patentansprüche

1. Verfahren zur Herstellung einer ein stabilen biologisch aktives Material umfassenden biologisch aktiven Substanz, bei dem man:
(a) das biologisch aktive Material in einer gelöste Stoffe umfassenden Lösung löst, wobei es sich bei den gelösten Stoffen um ein aus der aus anorganischen Salzen und Zuckern bestehenden Gruppe ausgewähltes niedermolekulares Molekül handelt, unter Erhalt einer biologisch aktiven Lösung, wobei die biologisch aktive Lösung eine Konzentration an biologisch aktivem Material von mindestens 5% und eine Konzentration an gelösten Stoffen von weniger als 5% der biologisch aktiven Lösung aufweist, wobei die Prozentzahlen jeweils auf das Gesamtgewicht der biologisch aktiven Lösung bezogen sind,
(b) die biologisch aktive Lösung an trockenen, nicht gelbildenden wasserlöslichen Polysaccharidpartikeln in einem Massenverhältnis von biologisch aktiver Lösung zu Polysaccharidpartikeln zwischen 0,25:1 und 1:1 adsorbiert, unter Bildung von biologisch aktiven beladenen Polysaccharidpartikeln, und
(c) die biologisch aktiven beladenen Polysaccharidpartikel unter Erhalt der stabilen biologisch aktiven Substanz trocknet.

2. Verfahren nach Anspruch 1, bei dem man weiterhin vor dem Adsorptionsschritt einen gelösten Stoff aus der biologisch aktiven Lösung entfernt.

3. Verfahren nach Anspruch 1, bei dem man weiterhin die stabile biologisch aktive Substanz mit einer nicht mischbaren organischen Flüssigkeit versetzt, unter Bildung einer stabilen giessbaren Suspension.

4. Verfahren nach Anspruch 1, wobei das biologisch aktive Material aus der aus Aminosäuren und deren Derivaten, Arzneimitteln, Peptiden, Enzymen, industriellen Enzymen, Vitaminen, Vitaminderivaten und -komplexen, Carotinen, Antioxidationsmitteln, entzündungshemmenden Substanzen, kosmetischen Ölen, antimikrobiellen Wirkstoffen, Peroxiden, Agrochemikalien und einer Mischung davon bestehenden Gruppe ausgewählt ist.

5. Verfahren nach Anspruch 1, wobei das Polysaccharid aus der aus Stärken, Cellulosen, Gummen und deren modifizierten und derivatisierten Verbindungen bestehenden Gruppe ausgewählt ist.

6. Verfahren nach Anspruch 1, wobei es sich bei dem Polysaccharid um Carrageen, Pektin, Guaran oder Carboxymethylcellulose handelt.

7. Verfahren nach Anspruch 1, wobei die stabile biologisch aktive Substanz das biologisch aktive Material in einer Menge von mindestens 1 Gew.-% und das Polysaccharid in einer Menge von 20 Gew.-% oder mehr umfasst, wobei die Prozentzahlen jeweils auf das Gesamtgewicht der biologisch aktiven Substanz bezogen sind.

8. Verfahren nach Anspruch 1, bei dem man weiterhin die stabile biologisch aktive Substanz mit einer Schicht geschmolzener fester Fette oder Wachse überzieht.

9. Verfahren nach Anspruch 1, wobei das Massenverhältnis der biologisch aktiven Lösung zu den Polysaccharidpartikeln 0,5:1 beträgt.

10. Stabile biologisch aktive Substanz, welche ein biologisch aktives Material in einer Menge im Bereich von 1 Gew.-% bis 40 Gew.-% und ein wasserlösliches Polysaccharid in einer Menge im Bereich von 1 Gew.-% bis 70 Gew.-% umfasst, wobei die stabile biologisch aktive Substanz durch das Verfahren nach Anspruch 1 hergestellt wird.

11. Stabile biologisch aktive Substanz nach Anspruch 10, weiterhin umfassend einen Emulgator oder Stabilisator in einer Menge im Bereich von 0,5 Gew.-% bis 10 Gew.-%, bezogen auf das Gesamtgewicht der biologisch aktiven Substanz.

12. Stabile biologisch aktive Substanz nach Anspruch 10, wobei es sich bei dem biologisch aktiven Material um ein Enzym ausgewählt aus der aus Proteasen, Lipasen, Amylasen, Cellulasen, Phosphatasen, Peroxidasen, Oxidasen und einer Mischung davon bestehenden Gruppe handelt.

13. Stabile biologisch aktive Substanz nach Anspruch 12, weiterhin umfassend eine Verbindung ausgewählt aus der aus natürlichen und synthetischen Antioxidationsmitteln, Metallionen, Alkalisilikaten, -carbonaten oder -hydrogencarbonaten, Sulfaten, Phosphaten, Boraten und Borsäure bestehenden Gruppe.

14. Stabile biologisch aktive Substanz nach Anspruch 13, wobei die Verbindung das Enzym stabilisiert.

15. Produkt, umfassend die stabile biologisch aktive Substanz nach Anspruch 10, wobei es sich bei dem biologisch aktiven Material um ein Enzym handelt.

## Revendications

1. Méthode de préparation d'une substance bioactive stable comprenant un matériau bioactif, comprenant :
(a) la solubilisation du matériau bioactif dans une solution comprenant des solutés, où les solutés sont constitués d'une petite molécule, choisie dans le groupe constitué par les sels inorganiques et les sucres, de façon à obtenir une solution bioactive, où la solution bioactive possède une concentration en matériau bioactif d'au moins 5% et une concentration en soluté inférieure à 5% de la solution bioactive, chaque pourcentage étant basé sur le poids total de la solution bioactive ;
(b) l'adsorption de la solution bioactive sur des particules sèches de polysaccharide hydrosoluble non gélifiant, selon un rapport massique de la solution bioactive aux particules de polysaccharide compris entre 0,25:1 et 1:1, de façon à former des particules de polysaccharide chargées et bioactives ; et
(c) le séchage des particules de polysaccharide chargées et bioactives, de façon à obtenir la substance bioactive stable.

2. Méthode selon la revendication 1, comprenant en outre l'élimination d'un soluté à partir de la solution bioactive préalablement à l'étape d'adsorption.

3. Méthode selon la revendication 1, comprenant en outre l'addition d'un liquide organique immiscible à la substance bioactive stable, de façon à former une suspension versable et stable.

4. Méthode selon la revendication 1, dans laquelle le matériau bioactif est choisi dans le groupe constitué par les acides aminés et leurs dérivés, les médicaments, les peptides, les enzymes, les enzymes industrielles, les vitamines, les dérivés et complexes de vitamines, les carotènes, les antioxydants, les substances anti-inflammatoires, les huiles cosmétiques, les ingrédients actifs antimicrobiens, les peroxydes, les substances agrochimiques et un mélange de ceux-ci.

5. Méthode selon la revendication 1, dans laquelle le polysaccharide est choisi dans le groupe constitué par les amidons, les celluloses, les gommes et leurs composés modifiés et dérivés.

6. Méthode selon la revendication 1, dans laquelle le polysaccharide est un carraghénane, une pectine, la gomme guar ou la carboxyméthylcellulose.

7. Méthode selon la revendication 1, dans laquelle la substance bioactive stable comprend le matériau bioactif selon une quantité d'au moins 1% en poids et le polysaccharide selon une quantité de 20% ou plus en poids, chaque pourcentage étant basé sur le poids total de la substance bioactive.

8. Méthode selon la revendication 1, comprenant en outre le revêtement de la substance bioactive stable par une couche de cires ou de matières grasses solides fondues.

9. Méthode selon la revendication 1, dans laquelle le rapport massique de la solution bioactive aux particules de polysaccharide est de 0,5:1.

10. Substance bioactive stable comprenant un matériau bioactif selon une quantité allant de 1% à 40% en poids et un polysaccharide hydrosoluble selon une quantité allant de 1% à 70% en poids, la substance bioactive stable étant produite par la méthode selon la revendication 1.

11. Substance bioactive stable selon la revendication 10, comprenant en outre un émulsifiant ou un stabilisant selon une quantité allant de 0,5% à 10% en poids, sur la base du poids total de la substance bioactive.

12. Substance bioactive stable selon la revendication 10, dans laquelle le matériau bioactif est une enzyme choisie dans le groupe constitué par les protéases, les lipases, les amylases, les cellulases, les phosphatases, les peroxydases, les oxydases et un mélange de celles-ci.

13. Substance bioactive stable selon la revendication 12, comprenant en outre un composé choisi dans le groupe constitué par les antioxydants naturels ou synthétiques, les ions métalliques, les silicates, carbonates ou bicarbonates, sulfates, phosphates, borates de métaux alcalins, et l'acide borique.

14. Substance bioactive stable selon la revendication 13, ce par quoi le composé stabilise l'enzyme.

15. Produit comprenant la substance bioactive stable selon la revendication 10, dans lequel le matériau bioactif est une enzyme.
